# EUROPEAN PATENT SPECIFICATION

(11) **EP 1 268 801 B1**
(45) Date of publication and mention of the grant of the patent: **23.06.2004**
(21) Application number: 01925538.9
(22) Date of filing: 04.04.2001
(51) Int. Cl.: C12N 15/13, C12N 15/62, C07K 16/46, C12N 15/10

(54) **A METHOD FOR IN VITRO MOLECULAR EVOLUTION OF ANTIBODY FUNCTION**
VERFAHREN ZUR MOLEKULAREN EVOLUTION DER ANTIKÖRPERFUNKTION
PROCEDE D'EVOLUTION MOLECULAIRE IN VITRO DE LA FONCTION D'ANTICORPS

(30) Priority: 05.04.2000 GB 0008419
(43) Date of publication of application: 02.01.2003
(73) Proprietor: Bioinvent International AB, 223 70 Lund (SE)
(72) Inventor: OHLIN, Mats, S-211 46 Malmo (SE); SODERLIND, Eskil, S-247 35 Sodra Sanby (SE); CARLSSON, Roland, S-226 54 Lund (SE)
(74) Representative: Thomas, Philip John Duval
(86) International application number: PCT/EP2001/004065
(87) International publication number: WO 2001/075091

(56) References cited:
- WO-A-98/32845
- JIRHOLT P. ET AL.: "Exploiting sequence space: shuffling in vivo formed complementarity determining regions into a master framework" GENE, vol. 215, no. 2, July 1998 (1998-07), pages 471-476, XP004149272 ISSN: 0378-1119 cited in the application
- KNAPPIK A. ET AL.: "Fully synthetic human combinatorial antibody libraries (HuCAL) based on modular consensus frameworks and CDRs randomized with trinucleotides" JOURNAL OF MOLECULAR BIOLOGY, vol. 296, no. 1, 11 February 2000 (2000-02-11), pages 57-86, XP000939143 ISSN: 0022-2836
- SÖDERLIND ET AL.: "Complementarity-determinig region (CDR) implantation: a theme of recombinantion" IMMUNOTECHNOLOGY, vol. 4, March 1999 (1999-03), pages 279-285, XP001053415 cited in the application

### SEQUENCE LISTING

<110> BioInvent International AB
   <120> A method for in vitro molecular evolution of antibody function
<130> BIOT/P24451PC
<140> PCT/EP01/04065
   <141> 4 April 2001
<150> GB 0008419.4
   <151> 5 April 2000
<160> 36
<170> SeqWin99
<210> 1
   <211> 36
   <212> DNA
   <213> Homo sapiens
<400> 1
   cgccaggctc cagggaaggg gctggagtgg gtggcc                                    36
<210> 2
   <211> 12
   <212> PRT
   <213> Homo sapiens
<400> 2
   Arg Gln Ala Pro Gly Lys Gly Leu Glu Trp Val Ala
   1               5                   10
<210> 3
   <211> 27
   <212> DNA
   <213> Homo sapiens
<400> 3
   cgattcacca tctccagaga caacccc                                              27
<210> 4
   <211> 9
   <212> PRT
   <213> Homo sapiens
<400> 4
   Arg Phe Thr Ile Ser Arg Asp Asn Ser
   1               5
<210> 5
   <211> 36
   <212> DNA
   <213> Homo sapiens
<400> 5
   cgccagatgc ccgggaaagg cctggagtgg gttggc                            36
<210> 6
   <211> 12
   <212> PRT
   <213> Homo sapiens
<400> 6
   Arg Gln Met Pro Gly Lys Gly Leu Glu Trp Val Gly
   1               5                   10
<210> 7
   <211> 27
   <212> DNA
   <213> Homo sapiens
<400> 7
   agattcacca tctcaagaga tgattca                                      27
<210> 8
   <211> 9
   <212> PRT
   <213> Homo sapiens
<400> 8
   Arg Phe Thr Ile Ser Arg Asp Asp Ser
   1               5
<210> 9
   <211> 36
   <212> DNA
   <213> Homo sapiens
<400> 9
   gcggtctacg ggccctttcc ggaccacacc caaccg 36
<210> 10
   <211> 27
   <212> DNA
   <213> Homo sapiens
<400> 10
   tctaagtggt agagttctct actaagt 27
<210> 11
   <211> 36
   <212> DNA
   <213> Artificial Sequence
<220>
   <223> PCR primer
<400> 11
   cgccagatgc ccgggaaagg cctggagtgg gttggc 36
<210> 12
   <211> 27
   <212> DNA
   <213> Artificial Sequence
<220>
   <223> PCR primer
<400> 12
   tctaagtggt agagttctct actaagt 27
<210> 13
   <211> 36
   <212> DNA
   <213> Artificial Sequence
<220>
   <223> PCR primer
<400> 13
   cgccaggctc cagggaaggg gctggagtgg gtggcc 36
<210> 14
   <211> 26
   <212> DNA
   <213> Artificial Sequence
<220>
   <223> PCR primer
<400> 14
   ctaagtggta gaggtctctg ttgggg 26
<210> 15
   <211> 27
   <212> DNA
   <213> Artificial Sequence
<220>
   <223> PCR product
<400> 15
   agattcacca tctccagaga caacccc 27
<210> 16
   <211> 42
   <212> DNA
   <213> Homo sapiens
<400> 16
   cgccaggctc cagggaaggg gctggagtgg gtggccgcta tt 42
<210> 17
   <211> 14
   <212> PRT
   <213> Homo sapiens
<400> 17
   Arg Gln Ala Pro Gly Lys Gly Leu Glu Trp Val Ala Asn Ile
   1               5                   10
<210> 18
   <211> 27
   <212> DNA
   <213> Homo sapiens
<400> 18
   cgattcacca tctccagaga caacccc 27
<210> 19
   <211> 9
   <212> PRT
   <213> Homo sapiens
<400> 19
   Arg Phe Thr Ile Ser Arg Asp Asn Ala
   1               5
<210> 20
   <211> 42
   <212> DNA
   <213> Homo sapiens
<400> 20
   cgccagatgc ccgggaaagg cctggagtgg atggggatca tc 42
<210> 21
   <211> 14
   <212> PRT
   <213> Homo sapiens
<400> 21
   Arg Gln Met Pro Gly Lys Gly Leu Glu Trp Met Gly Ile Ile
   1 5 10
<210> 22
   <211> 27
   <212> DNA
   <213> Homo sapiens
<400> 22
   caggtcacca tctcagccga caagtcc 27
<210> 23
   <211> 9
   <212> PRT
   <213> Homo sapiens
<400> 23
   Gln Val Thr Ile Ser Ala Asp Lys Ser
   1 5
<210> 24
   <211> 42
   <212> DNA
   <213> Artificial Sequence
<220>
   <223> PCR primer
<400> 24
   cgccagatgc ccgggaaagg cctggagtgg atggggatca tc 42
<210> 25
   <211> 27
   <212> DNA
   <213> Artificial Sequence
<220>
   <223> PCR primer
<400> 25
   gtccagtggt agagtcggct gttcagg 27
<210> 26
   <211> 42
   <212> DNA
   <213> Homo sapiens
<400> 26
   gcggtctacg ggccctttcc ggacctcacc tacccctagt ag 42
<210> 27
   <211> 27
   <212> DNA
   <213> Homo sapiens
<400> 27
   gtccagtggt agagtcggct gttcagg 27
<210> 28
   <211> 41
   <212> DNA
   <213> Artificial Sequence
<220>
   <223> PCR primer
<400> 28
   cgccaggctc cagggaaggg gctggagtgg gtggcggcca t 41
<210> 29
   <211> 27
   <212> DNA
   <213> Artificial Sequence
<220>
   <223> PCR primer
<400> 29
   gcccagtggt agaggtctct gttgggg 27
<210> 30
   <211> 41
   <212> DNA
   <213> Artificial Sequence
<220>
   <223> PCR primer
<400> 30
   cgccaggctc cagggaaggg gctggagtgg gtggccgcta t 41
<210> 31
   <211> 27
   <212> DNA
   <213> Artificial Sequence
<220>
   <223> PCR primer
<400> 31
   gctaagtggt agaggtctct gttgggg 27
<210> 32
   <211> 42
   <212> DNA
   <213> Artificial Sequence
<220>
   <223> PCR product
<400> 32
   cgccaggctc cagggaaggg gctggagtgg gtggcggcca tc 42
<210> 33
   <211> 27
   <212> DNA
   <213> Artificial Sequence
<220>
   <223> PCR product
<400> 33
   cgggtcacca tctccagaga caactcc 27
<210> 34
   <211> 42
   <212> DNA
   <213> Artificial Sequence
<220>
   <223> PCR product
<400> 34
   gcggtccgag gtcccttccc cgacctcacc cacgcgcggt ag 42
<210> 35
   <211> 27
   <212> DNA
   <213> Artificial Sequence
<220>
   <223> PCR product
<400> 35
   gcccagtggt agaggtctct gttgagg 27
<210> 36
   <211> 42
   <212> DNA
   <213> Artificial Sequence
<220>
   <223> PCR product
<400> 36
   cgccaggctc cagggaaggg gctggagtgg gtggccgcta tc 42

## Description

### Field of the invention

The present invention relates to a method for *in vitro* molecular evolution of antibody function.

### Background of the invention

WO98/32845, Söderlind *et al.* (1999), and Jirholt *et al.* (1998) describe the *in vitro* molecular evolution of antibody-derived proteins, by implanting naturally occurring complementarity determining regions (CDRs) into a defined and selected ("master") framework, comprising the framework regions from the germline gene DP-47 of the V_{H}3 family. Oligonucleotide primers, based on the sequences encoding parts of the framework regions of DP-47 immediately flanking the CDRs, are used to amplify nucleic acid sequences encoding CDRs from a cDNA library prepared from peripheral blood B cells. Single stranded DNA from the amplification reaction is then combined with overlapping oligonucleotides which encode the remainder of the master framework in an overlap extension PCR reaction to produce full length sequences encoding V_{H} antibody domains which each contain the framework regions of the DP-47 germline gene, and CDRs from germline genes of the V_{H}3 family.

This technique provides a valuable means of increasing diversity in antibody libraries (e.g. phage display libraries), particularly as it allows recombination of CDR1 and CDR2, which are normally linked *in vivo* and do not undergo recombination during germline gene rearrangement.

Moreover, the CDRs have been proof-read *in vivo* and are unlikely to be immunogenic, providing an advantage over artificially mutated CDR sequences. Also, the master frameworks utilised (from germline DP-47 and DPL3) were selected to be highly compatible with the bacterial expression, and phage system employed, thus ensuring a high degree of functional protein display.

Another important aspect in the construction of the library was a demand on the framework to be able to hold specificities, with high affinities, against a large variety of different types of antigens. As the system allows variability to be introduced into any number of the CDR positions, the achievable variability is huge, far beyond what can be obtained by previously established combinatorial technologies. Finally, the modular design, *i.e.* the fact that variability is introduced into a common framework structure, makes subsequent modifications and studies of selected clones simple and efficient.

Using this technology, tentatively called CDR-implantation, a large phage display antibody library based on the single chain Fv (scFv) format has been created. The library has been used to select a panel of high affinity antibodies against a number of ligand types, including proteins (of human and non-human origin), peptides, carbohydrates and low-molecular weight haptens. Thus, from a functional point of view, it is clear that a single, selected, master framework can hold antibody specificities with high affinities against quite different types of antigens, suggesting that the topology of the surfaces may differ greatly between antibodies.

However, there remains a general need in the art to increase the diversity of antibody libraries.

### Summary of the invention

According to the present invention, nucleic acid encoding a CDR that is normally contained in a framework (the "original framework"), which differs from a selected master framework, is amplified from an immunoglobulin gene and is inserted into nucleic acid encoding the selected master framework.

Amplification may be accomplished, as with conventional CDR implantation as described above, by PCR using primers based on the framework regions flanking the CDRs. However, in the present invention, the original framework and the master framework differ. In contrast with the previously described CDR implantation methods, therefore, nucleic acid encoding the CDRs is not amplified using primers based exclusively on the master framework. Rather, primers are used which differ from the sequence encoding the master framework. The primers may be based specifically on a particular other framework, *e.g.* that of a different germline gene or consensus sequence of a germline gene family, or may be degenerate, *e.g.* to amplify CDRs from a range of germline families.

Accordingly, in a first aspect, the present invention provides a method for producing a polynucleotide sequence encoding an antibody variable domain, the variable domain comprising complementarity-determining regions (CDRs) located within a selected framework (the 'master framework'), the method comprising the steps of:
a) providing at least one nucleic acid molecule encoding one or more CDRs and associated framework regions (the 'original framework');
b) amplifying at least one CDR-encoding portion of the nucleic acid molecule(s) of step (a) using one or more pairs of oligonucleotides as amplification primers and;
c) assembling a polynucleotide sequence encoding an antibody variable domain by combining the amplified CDR-encoding nucleotide sequences produced in step (b) with nucleotide sequences encoding said master framework,
wherein the oligonucleotide primers of step (b) comprise nucleotide sequences which differ from the corresponding nucleotide sequences encoding said master framework.

A second aspect of the invention provides a method for producing a library of polynucleotide sequences each encoding an antibody variable domain comprising complementarity-determining regions (CDRs) located within a common selected framework (the 'master framework'), the method comprising the steps of:
a) providing a population of nucleic acid molecules encoding one or more complementarity-determining regions (CDRs) and associated framework regions (the 'original framework');
b) amplifying at least one CDR-encoding portion of the nucleic acid molecules of step (a) using one or more pairs of oligonucleotides as amplification primers and;
c) assembling a polynucleotide sequence encoding an antibody variable domain by combining the amplified CDR-encoding nucleotide sequences produced in step (b) with nucleotide sequences encoding said master framework,
wherein the oligonucleotide primers of step (b) comprise nucleotide sequences which differ from the corresponding nucleotide sequences encoding said master framework.

By "associated framework regions", as used in relation to the nucleic acid molecules provided in step (a), we mean the amino acid residues of the framework region immediately flanking the CDR. For example, the nucleic acid molecule(s) of step (a) may encode a CDR together with up to 5, 10, 15 or more amino acid residues of the framework flanking either side of the CDR. Thus, the nucleic acid molecule(s) of step (a) may encode an antibody variable region or even an entire antibody.

By "differ from", in the context of the nucleotide sequences of the oligonucleotide primers of step (b), we mean that the regions of the oligonucleotide primers of step (b) which encode framework residues (*i.e.* those regions of the oligonucleotide primers which are complementary to regions of the nucleic acid molecules provided in step (a) which encode framework residues) do not share 100% sequence identity with the corresponding regions of the nucleic acid molecules encoding the master framework.

In a preferred embodiment of the first and second aspects of the invention, the method comprises the steps of:
i) providing at least one pair of oligonucleotides;
ii) using each said pair of oligonucleotides as amplification primers to amplify nucleotide sequences encoding different CDRs, and;
iii) assembling polynucleotide sequences encoding antibody variable domains by incorporating nucleotide sequences derived from step ii) above with nucleotide sequences encoding framework sequences (FRs) of a selected type,
wherein the oligonucleotides of step (i) have sequences which differ from corresponding sequences encoding said master framework.

For the avoidance of doubt, the "framework" of a variable region, as used herein, is typically made up of four individual framework regions, which flank the three CDRs of the variable region:
[---FR1---] [CDR1] [---FR2---] [CDR2] [---FR3---] [CDR3] [---FR4---]

The "framework regions (FRs) of a selected type" together provide a "master framework".

It will be appreciated by persons skilled in the art that the methods of the present invention may be used to produce a polynucleotide sequence encoding a variable domain of different types of antibody. For example, the variable domain may be an IgG, IgM, IgA, IgD or IgE variable domain.

Preferably, the polynucleotide sequence assembled in step (c) encodes an IgG variable domain. Advantageously, the polynucleotide sequence(s) assembled in step (c) encodes an IgG heavy chain or light chain. Conveniently, the polynucleotide sequence(s) assembled in step (c) encodes a non-naturally occurring antibody variable domain.

Advantageously, the polynucleotide sequence(s) assembled in step (c) encodes an antibody variable domain comprising at least one CDR having a canonical structure which is atypical of antibody variable domains comprising the master framework. By 'atypical' we mean that the CDR has a canonical structure which is found in less than 10% of naturally-occurring antibody variable domains comprising the selected master framework. Preferably, the CDR has a canonical structure which is found in less than 5%, 2% or 1% of naturally-occurring antibody variable domains comprising the selected master framework. Most preferably, the CDR has a canonical structure which is not found in any naturally-occurring antibody variable domains comprising the selected master framework.

In a preferred embodiment of the methods of the invention, at least one of the polynucleotide sequence(s) assembled in step (c) encode an antibody variable domain comprising at least one CDR derived a different germline gene family to that of the master framework. For example, the polynucleotide sequence(s) assembled in step (c) may encode an antibody variable domain comprising one or more CDRs derived from a light chain in heavy chain framework, or vice versa.

Advantageously, step (a) comprises providing a population of nucleic acid molecules each encoding an antibody variable domain. Conveniently, the nucleic acid molecules each encode an antibody variable domain from the same germline gene family. Thus, selectivity for the CDRs to be incorporated into the master framework may be achieved, at least in part, by providing a chosen population of nucleic acid molecules in step (a).

Alternatively, or in addition, selectivity for the CDRs to be incorporated into the master framework may be achieved by using oligonucleotide primer pairs in step (b) which selectively hybridise to a target sub-population of nucleic acid molecules provided in step (a). By 'selectively hybridise' we mean that the oligonucleotide primer pairs hybridise selectively to a target sub-population of nucleic acid molecules under conditions of high stringency. Oligonucleotide hybridisation conditions are described in *Molecular Cloning: A Laboratory Manual* (third edition), Sambrook and Russell (eds.), Cold Spring Harbor Laboratory Press.

It will be appreciated by persons skilled in the art that the ability of the primers to selectively hybridise with target nucleic acid molecules is dependent, to a large extent, on the degree of sequence complementarity between the primers and the target sequences.

Preferably, the oligonucleotide primer pairs in step (b) selectively hybridise to a target sub-population of nucleic acid molecules provided in step (a) each encoding an antibody variable domain from the same germline gene family.

Thus, by providing a chosen population of nucleic acid molecules in step (a) and/or by using oligonucleotide primer pairs in step (b) selectively hybridise to a target sub-population of nucleic acid molecules provided in step (a), it is possible to select the CDRs to be incorporated into the master framework.

In a preferred embodiment, the CDRs to be incorporated into the master framework are derived from nucleic acid molecules encoding an antibody variable domain from the same germline gene family, such as the V_{H}3 family. Conveniently, the CDRs to be incorporated into the master framework are derived from nucleic acid molecules encoding an antibody variable domain from the same germline gene, such asDP-29 and DP-73.

Advantageously, the master framework is derived from a germline gene selected from the group consisting of DP-47 and DPL-3.

In a preferred embodiment of the methods of the invention, step (c) comprises the use of overlap extension PCR (see Sambrook & Russell, *supra).* In this case, it is necessary to isolate single stranded nucleic acid molecules from the amplified CDR-encoding nucleic acid molecules produced in step (b). This may be achieved by using oligonucleotide primer pairs in which one of the primers is biotinylated, thereby enabling the nucleic acid strand produced by extension of the biotinylated primer to be isolated on the basis of its affinity for streptavidin. The use of biotinylated primers and overlap extension PCR is described in Jirholt *et al,* 1998, *supra.*

As a consequence of the differences in the nucleotide sequence between the regions of the amplification primers which encode framework residues and the nucleic acid sequences encoding the corresponding regions of the master framework, the amplified CDRs cannot always be incorporated directly into the master framework by overlap extension PCR since the nucleotides encoding the amplified CDRs may fail adequately to anneal with the nucleic acid encoding the master framework. This is particularly the case when (as in Example 1B) there are significant mismatches at the ends of the amplification primers (notably at the end nearer the CDR). As a result, it may be necessary to alter the regions of the amplified nucleotide sequences which encode the framework regions flanking the CDRs to make them more similar in sequence to the regions of the nucleic acid molecules encoding the corresponding regions of the master framework.

Thus, in a preferred embodiment of the methods of the invention, the methods comprise a further step, performed after step (b) and prior to step (c), of modifying the nucleotide sequence of the amplified CDR-encoding molecules of step (b) such that the portions of said amplified molecules which encode framework regions share greater sequence identity with the corresponding portions of the master framework. Preferably the nucleotide sequences are modified such that the portions of said amplified molecules which encode framework regions share 100% sequence identity with the corresponding portions of the master framework.

For example, one way of accomplishing this is to initially amplify the CDR and adjacent portions of the flanking framework regions using PCR primers which are identical or very similar to the original framework of the CDR. Then, in successive rounds of PCR amplification, one can modify the regions of the amplified nucleic acid sequences which encode framework regions using primers containing mismatches relative to the original framework, those mismatches providing the residues present at corresponding positions in the master framework. Eventually, the framework regions become sufficiently similar, or identical, to the master framework to be incorporated therein by overlap extension PCR.

Alternatively, a single round of PCR amplification using primers that represent a chimaera of the original and master frameworks may suffice to amplify CDRs which can be used in the overlap extension PCR process, in which case the additional step is not required.

In the first round of PCR (Step 'b'), therefore, irrespective of whether said additional steps are to be performed, it may be preferable to include mismatches in the primers relative to the original framework, in order to include as many bases as possible that are common to the selected master framework. The number of mismatches that can be included depends on a set of factors including the number of bases that differ between the frameworks, the length of the primers and the risk of annealing to sequences other than those intended.

It may be possible for CDRs amplified by primers which are identical to the original framework to be incorporated directly into the master framework (i.e. the products of step 'b'), by overlap extension PCR. Alternatively, inclusion of additional PCR steps (as described above) may be necessary to make the framework sequences associated with the amplified CDR-encoding more similar to the corresponding sequences in the master framework.

In a preferred embodiment of the first aspect of the invention, the method comprising a further step of inserting the polynucleotide sequence(s) assembled in step (c) into an expression vector. Advantageously, the expression vector is a secretion vector.

Thus, polynucleotide sequences produced by the methods of the invention may be used in accordance with known techniques, appropriately modified in view of the teachings contained herein, to construct an expression vector, which is then used to transform an appropriate host cell for the expression and production of antibody variable domains. Such techniques include those disclosed in US Patent Nos. 4,440,859 issued 3 April 1984 to Rutter *et al,* 4,530,901 issued 23 July 1985 to Weissman, 4,582,800 issued 15 April 1986 to Crowl, 4,677,063 issued 30 June 1987 to Mark *et al,* 4,678,751 issued 7 July 1987 to Goeddel, 4,704,362 issued 3 November 1987 to Itakura *et al,* 4,710,463 issued 1 December 1987 to Murray, 4,757,006 issued 12 July 1988 to Toole, Jr. *et al,* 4,766,075 issued 23 August 1988 to Goeddel *et al* and 4,810,648 issued 7 March 1989 to Stalker, all of which are incorporated herein by reference.

The polynucleotide sequences produced by the methods of the invention may be joined to a wide variety of other DNA sequences for introduction into an appropriate host. The companion DNA will depend upon the nature of the host, the manner of the introduction of the DNA into the host, and whether episomal maintenance or integration is desired.

Generally, the polynucleotide sequence is inserted into an expression vector, such as a plasmid, in proper orientation and correct reading frame for expression. If necessary, the polynucleotide sequence may be linked to the appropriate transcriptional and translational regulatory control nucleotide sequences recognised by the desired host, although such controls are generally available in the expression vector. Thus, the polynucleotide sequence insert may be operatively linked to an appropriate promoter. Bacterial promoters include the *E.coli lacI* and *lacZ* promoters, the T3 and T7 promoters, the *gpt* promoter, the phage λ PR and PL promoters, the *phoA* promoter and the *trp* promoter. Eukaryotic promoters include the CMV immediate early promoter, the HSV thymidine kinase promoter, the early and late SV40 promoters and the promoters of retroviral LTRs. Other suitable promoters will be known to the skilled artisan. The expression constructs will desirably also contain sites for transcription initiation and termination, and in the transcribed region, a ribosome binding site for translation (Hastings *et al,* International Patent No. WO 98/16643, published 23 April 1998).

The vector is then introduced into the host through standard techniques. Generally, not all of the hosts will be transformed by the vector and it will therefore be necessary to select for transformed host cells. One selection technique involves incorporating into the expression vector a DNA sequence marker, with any necessary control elements, that codes for a selectable trait in the transformed cell. These markers include dihydrofolate reductase, G418 or neomycin resistance for eukaryotic cell culture, and tetracyclin, kanamycin or ampicillin resistance genes for culturing in *E.coli* and other bacteria. Alternatively, the gene for such selectable trait can be on another vector, which is used to co-transform the desired host cell.

Host cells that have been transformed by the expression vector are then cultured for a sufficient time and under appropriate conditions known to those skilled in the art in view of the teachings disclosed herein to permit the expression of the encoded antibody variable domain, which can then be recovered.

The antibody variable domain can be recovered and purified from recombinant cell cultures by well-known methods including ammonium sulphate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography ("HPLC") is employed for purification.

Many expression systems are known, including systems employing: bacteria (*e.g. E. coli* and *Bacillus subtilis*) transformed with, for example, recombinant bacteriophage, plasmid or cosmid DNA expression vectors; yeasts (*e.g. Saccaromyces cerevisiae*) transformed with, for example, yeast expression vectors; insect cell systems transformed with, for example, viral expression vectors (*e.g.* baculovirus); plant cell systems transfected with, for example viral or bacterial expression vectors; animal cell systems transfected with, for example, adenovirus expression vectors.

The vectors may include a prokaryotic replicon, such as the Col El *ori,* for propagation in a prokaryote, even if the vector is to be used for expression in other, non-prokaryotic cell types. The vectors may also include an appropriate promoter such as a prokaryotic promoter capable of directing the expression (transcription and translation) of the genes in a bacterial host cell, such as *E.coli,* transformed therewith.

A promoter is an expression control element formed by a DNA sequence that permits binding ofRNA polymerase and transcription to occur. Promoter sequences compatible with exemplary bacterial hosts are typically provided in plasmid vectors containing convenient restriction sites for insertion of a DNA segment of the present invention.

Typical prokaryotic vector plasmids are: pUC18, pUC19, pBR322 and pBR329 available from Biorad Laboratories (Richmond, CA, USA); pTrc99A, pKK223-3, pKK233-3, pDR540 and pRIT5 available from Pharmacia (Piscataway, NJ, USA); pBS vectors, Phagescript vectors, Bluescript vectors, pNH8A, pNH16A, pNH18A, pNH46A available from Stratagene Cloning Systems (La Jolla, CA 92037, USA).

A typical mammalian cell vector plasmid is pSVL available from Pharmacia (Piscataway, NJ, USA). This vector uses the SV40 late promoter to drive expression of cloned genes, the highest level of expression being found in T antigen-producing cells, such as COS-1 cells. An example of an inducible mammalian expression vector is pMSG, also available from Pharmacia (Piscataway, NJ, USA). This vector uses the glucocorticoid-inducible promoter of the mouse mammary tumour virus long terminal repeat to drive expression of the cloned gene.

Useful yeast plasmid vectors are pRS403-406 and pRS413-416 and are generally available from Stratagene Cloning Systems (La Jolla, CA 92037, USA). Plasmids pRS403, pRS404, pRS405 and pRS406 are Yeast Integrating plasmids (YIps) and incorporate the yeast selectable markers *HIS3, TRP1, LEU2* and *URA3.* Plasmids pRS413-416 are Yeast Centromere plasmids (YCps).

Methods well known to those skilled in the art can be used to construct expression vectors containing the coding sequence and, for example appropriate transcriptional or translational controls. One such method involves ligation via homopolymer tails. Homopolymer polydA (or polydC) tails are added to exposed 3' OH groups on the DNA fragment to be cloned by terminal deoxynucleotidyl transferases. The fragment is then capable of annealing to the polydT (or polydG) tails added to the ends of a linearised plasmid vector. Gaps left following annealing can be filled by DNA polymerase and the free ends joined by DNA ligase.

Another method involves ligation via cohesive ends. Compatible cohesive ends can be generated on the DNA fragment and vector by the action of suitable restriction enzymes. These ends will rapidly anneal through complementary base pairing and remaining nicks can be closed by the action of DNA ligase.

A further method uses synthetic molecules called linkers and adaptors. DNA fragments with blunt ends are generated by bacteriophage T4 DNA polymerase or *E.coli* DNA polymerase I which remove protruding 3' termini and fill in recessed 3' ends. Synthetic linkers, pieces of blunt-ended double-stranded DNA which contain recognition sequences for defined restriction enzymes, can be ligated to blunt-ended DNA fragments by T4 DNA ligase. They are subsequently digested with appropriate restriction enzymes to create cohesive ends and ligated to an expression vector with compatible termini. Adaptors are also chemically synthesised DNA fragments which contain one blunt end used for ligation but which also possess one preformed cohesive end.

Synthetic linkers containing a variety of restriction endonuclease sites are commercially available from a number of sources including International Biotechnologies Inc, New Haven, CN, USA.

A desirable way to modify the DNA encoding the polypeptide of the invention is to use the polymerase chain reaction as disclosed by Saiki *et al* (1988) *Science* **239**, 487-491. In this method the DNA to be enzymatically amplified is flanked by two specific oligonucleotide primers which themselves become incorporated into the amplified DNA. The said specific primers may contain restriction endonuclease recognition sites which can be used for cloning into expression vectors using methods known in the art.

The display of proteins and polypeptides on the surface of bacteriophage (phage), fused to one of the phage coat proteins, provides a powerful tool for the selection of specific ligands. This 'phage display' technique was originally used by Smith in 1985 (*Science* **228**, 1315-7) to create large libraries of antibodies for the purpose of selecting those with high affinity for a particular antigen. More recently, the method has been employed to present peptides, domains of proteins and intact proteins at the surface of phages in order to identify ligands having desired properties.

The principles behind phage display technology are as follows:
(i) Nucleic acid encoding the protein or polypeptide for display is cloned into a phage;
(ii) The cloned nucleic acid is expressed fused to the coat-anchoring part of one of the phage coat proteins (typically the p3 or p8 coat proteins in the case of filamentous phage), such that the foreign protein or polypeptide is displayed on the surface of the phage;
(iii) The phage displaying the protein or polypeptide with the desired properties is then selected (*e.g.* by affinity chromatography) thereby providing a genotype (linked to a phenotype) that can be sequenced, multiplied and transferred to other expression systems.

Alternatively, the foreign protein or polypeptide may be expressed using a phagemid vector (*i.e.* a vector comprising origins of replication derived from a phage and a plasmid) that can be packaged as a single stranded nucleic acid in a bacteriophage coat. When phagemid vectors are employed, a "helper phage" is used to supply the functions of replication and packaging of the phagemid nucleic acid. The resulting phage will express both the wild type coat protein (encoded by the helper phage) and the modified coat protein (encoded by the phagemid), whereas only the modified coat protein is expressed when a phage vector is used.

Methods of selecting phage expressing a protein or peptide with a desired specificity are known in the art. For example, a widely used method is "panning", in which phage stocks displaying ligands are exposed to solid phase coupled target molecules, *e.g.* using affinity chromatography.

Alternative methods of selecting phage of interest include SAP (Selection and Amplification of Phages; as described in WO 95/16027) and SIP (Selectively-Infective Phage; EP 614989A, WO 99/07842), which employ selection based on the amplification of phages in which the displayed ligand specifically binds to a ligand binder. In one embodiment of the SAP method, this is achieved by using non-infectious phage and connecting the ligand binder of interest to the N-terminal part of p3. Thus, if the ligand binder specifically binds to the displayed ligand, the otherwise non-infective ligand-expressing phage is provided with the parts of p3 needed for infection. Since this interaction is reversible, selection can then be based on kinetic parameters (see Duenas *et al.,* 1996, *Mol. Immunol.* **33**, 279-285).

The use of phage display to isolate ligands that bind biologically relevant molecules has been reviewed in Felici *et al.* (1995) *Biotechnol. Annual Rev.* **1**, 149-183, Katz (1997) *Annual Rev. Biophys. Biomol. Struct.* **26**, 27-45 and Hoogenboom *et al.* (1998) *Immunotechnology* 4(**1**), 1-20. Several randomised combinatorial peptide libraries have been constructed to select for polypeptides that bind different targets, *e.g.* cell surface receptors or DNA (reviewed by Kay, 1995, *Perspect. Drug Discovery Des.* **2**, 251-268; Kay and Paul, 1996, *Mol. Divers.* **1**, 139-140). Proteins and multimeric proteins have been successfully phage-displayed as functional molecules (see EP 0349578A, EP 4527839A, EP 0589877A; Chiswell and McCafferty, 1992, *Trends Biotechnol.* **10**, 80-84). In addition, functional antibody fragments (*e.g.* Fab, single chain Fv [scFv]) have been expressed (McCafferty *et al.,* 1990, *Nature* **348**; 552-554; Barbas *et al.,* 1991, *Proc. Natl. Acad Sci. USA* **88**, 7978-7982; Clackson *et al.,* 1991, *Nature* **352**, 624-628), and some of the shortcomings of human monoclonal antibody technology have been superseded since human high affinity antibody fragments have been isolated (Marks *et al.,*1991, *J. Mol. Biol.* **222**, 581-597; Hoogenboom and Winter, 1992, *J. Mol. Biol.* **227**, 381-388). Further information on the principles and practice ofphage display is provided in *Phage display of peptides and proteins: a laboratory manual* Ed Kay, Winter and McCafferty (1996) Academic Press, Inc ISBN 0-12-402380-0, the disclosure of which is incorporated herein by reference.

Thus, in a preferred embodiment of the first and second aspects of the invention, the method further comprises the step of expressing the polynucleotide sequence(s) assembled in step (c) and screening the resultant polypeptide(s), comprising an antibody variable domain, for desired properties. Preferably, the desired properties are readily selectable by known techniques. For example, antibodies may be screened for desired affinity using affinity chromatographic methods.

### Detailed description of the invention

The present invention represents a development of the technology presented in WO98/32845, Söderlind *et al.* (1999) *Immunotechnology* **4**, 279-285, and Jirholt *et al.* (1998) *Gene* **215**, 471-476, all of which are incorporated herein in their entirety, particularly for the purpose of describing generally the methods and conditions used for amplifying CDRs from a cDNA library containing antibody-encoding sequences, and methods, materials and conditions for reassembling the CDRs thus amplified into the master framework by overlap extension PCR.

The method may further comprise the step of expressing the resulting antibody encoded by the assembled nucleotide sequence and screening for desired properties. Again, this is described in detail in the above-mentioned references.

The resulting expressed antibody can be screened for desired characteristics. For example it may be desirable to alter its ability to specifically bind to an antigen or to improve its binding properties in comparison to the parent antibody. Once more, this is described in detail in the above-mentioned references.

Preferably the oligonucleotides used for amplification primers have at least two nucleic acid residues different from a corresponding portion of the nucleic acid sequence encoding the master framework. More preferably there are at least 3, 4, 5, 6, 7, 8, 10 or 12 different nucleic acid residues. In an alternative definition, the amplification primers preferably have no more than about 95% sequence identity with a corresponding portion of the nucleic acid sequence encoding the master framework, more preferably no more than about 90%, 85%, 80%, 70% or 60% sequence identity.

In conventional CDR implantation, the amplification primers may include a small number of nucleotides encoding one or more amino acid residues of the adjoining end of the CDR (*e.g.* three nucleotides, encoding one CDR residue). This applies also to the present invention, and in such cases, the nucleotides of the CDR may be discounted when determining the number of nucleotide differences between the primer and the master framework.

Bearing in mind the teaching herein, and given in the cited references on the basic CDR-implantation technique, the skilled person will be able to design primers for amplifying the CDRs and, if necessary or desired, for modifying the amplification products to make their framework regions more similar to the selected master framework.

Where a particular germline gene is to be targeted, highly specific primers may be desired, for example based closely on the sequence encoding the parts of the framework regions of that gene which flank the CDR or CDRs to be amplified. The sequences of different germline genes are available from the VBASE sequence directory (URL: http://www.mrc-cpe.cam.ac.uk/imt-doc/public/INTRO.html) or from the DNAplot directory (URL: http://www.genetik.uni-koeln.de:80/dnaplot/vsearch human. html).

Similarly, the primers can be designed to amplify CDRs from a particular germline gene family, by designing primers based on the consensus sequence of genes of that family. For example, a consensus sequence can be defined as the sequence of bases found at >90% of loci of a particular germline family. Such sequences may include degenerate sites, indicating that different individual sequences have different nucleotides at that site. There may nevertheless be some common feature of the nucleotide residues which appear at such a degenerate site; such sites are designated R (purine; bases G and A), Y (pyrimidine; C, T), M (amino; A, C), K (keto; T, G), S (strong; C, G), W (weak, A, T), B (not A), D (not C), H (not G) or V (not T). A site where no common feature is evident is designated N (any).

Primers based on consensus sequences including such designations may be degenerate, *i.e.* a population of primers is made to include all possible combinations consistent with the consensus sequence, or where appropriate artificial bases which mimic particular sets of bases may be included within a homogeneous population of primers.

Information ascribing germline genes to germline gene families (such as the variable heavy germline gene families V_{H}1, V_{H}2, V_{H}3, V_{H}4, V_{H}5, V_{H}6 and V_{H}7) is available from the VBASE directory referred to above.

Similarly, it is possible also to design the primers to amplify CDRs from a plurality of germline gene families, using a consensus sequence of germline genes from said plurality of families. However, it will generally be preferred to target a particular germline gene or family.

With this in mind, the skilled person will be able to design appropriate primers depending on the specificity required. Preferably at least one primer of the or each pair used to initially amplify the CDRs is at least 15 nucleotides in length, more preferably at least 18, still more preferably at least 21 or 24, optionally at least 30, 36 or 42. Preferably, however, the primer is no more than 42 nucleotides in length, more preferably no more than 36 or 30, more preferably no more than 27.

Preferably the method will be used to implant CDRs at all three positions in the variable domain, since this leads to maximum variability, and ultimately more useful libraries. However, the method is not limited to this, and if desired (for example to optimise a previously obtained antibody), the method may be used to implant only one or two CDRs. In such cases, nucleic acid encoding the invariant CDR(s) will be included in the overlap extension PCR step, in addition to the newly amplified CDRs and the nucleic acid encoding the selected master framework.

The present invention is not to be construed as limited to implanting CDRs from immunoglobulin genes of the same general type as the master framework (*e.g.* implanting V_{H} CDRs into V_{H} master framework), although this is a preferred embodiment of the invention. Rather, the invention in its broader aspects includes the implantation of CDR-encoding nucleic acid from any type of immunoglobulin gene which has a variable region as defined above into a master framework which is independently of any such type of immunoglobulin superfamily gene. For example, Vλ CDRs may be inserted into a V_{H} master framework and *vice versa.* Moreover, any members of the immunoglobulin superfamily having analogous structures to CDRs and FRs may provide the CDRs and/or master FRs of the invention, the above description being applicable *mutatis mutandis.*

The term "antibody" is used herein in its broadest sense, to include also antibody fragments having a variable domain which includes CDRs flanked by framework regions. Examples of antibody fragments having such variable domains are the Fab fragment consisting of the V_{L}, V_{H}, C_{L} and C_{H}1 domains; the Fd fragment consisting of the V_{H}, and C_{H}1 domains; the Fv fragment consisting of the V_{L} and V_{H} domains of a single arm of an antibody; the dAb fragment which consists of a V_{H} domain; and the F(ab')2 fragment, a bivalent fragment including two Fab fragments linked by a disulphide bridge at the hinge region. Single chain Fv fragments are also included.

Any desired master framework regions (or "framework regions (FRs) of a selected type") may be utilised in the present invention. In particular, they may be selected to be highly compatible with the bacterial expression system, and phage system, to be employed, thus ensuring a high degree of functional protein display. Favoured examples are framework regions from the DP-47 and DPL-3 germline genes (of the V_{H}3 and Vλ germline gene families, respectively).

It is now generally agreed that the CDR-loops, which build up the surfaces of antibody combining sites, can be grouped into a limited number of so-called canonical structures, depending on their conformation after folding. The pioneering work in this area was performed by Cothia and Lesk (1987) who classified CDR 1 and 2 in the heavy chain and CDR 1-3 in the light chain into a few basic structures.

The concept of canonical structures is the result of extensive analyses of empirically determined and analysed antibody structures. The determinants for the canonical conformations are the lengths of the loops, key residues in the loops and key residues in the adjacent framework sequences (Chothia et al. 1992; Tomlinson *et al.* 1995; Al-Lazikani *et al.* 1997). For example, the human Vκ sequences can be grouped in 6 canonical structures for the CDRL 1 loop, 1 canonical structure for the CDRL2 loop and 5 canonical structures for the CDRL3 loop. Similarly, the human V_{H} sequences can be grouped in 3 canonical structures for the CDRH1 loop and 4 canonical structures for the CDRH2 loop.

The CDRH3 loop has not yet been classified in distinct canonical classes, most probably due to its inherited length variation which leads to unique properties regarding flexibility. However, recently it was demonstrated that this CDR also is built from structure elements forming a basic torso near, and to some extent including, the framework region, and an apical head region that sometimes includes an additional shoulder (Morea *et al.* 1998).

It is conceivable that nature has developed different types of canonical structures to deal with the multitude of antigenic structures the immune systems may encounter. Nature also presents these structures in the context of different framework structures. Thus, a particular CDR-loop is found in combination with a certain framework (VBASE). There also seems to be a bias to which canonical structures are used in order to create suitable surfaces, complementary to different types of antigens. In particular, loops with canonical structures building up a flat surface seem to yield surfaces that bind well to large protein antigens. These loops have a propensity to be rather short whereas longer loops are preferentially found in antibodies specific for smaller molecules *e.g.* haptens (Lara-Ochoa *et al.* 1996). Not all loops seem to be equally important in creating variability in the surfaces. Of course H3 is of major importance in this respect but also H2 and L1 determine the surfaces to a great extent (Vargas-Madrazo *et al.* 1995).

Using the CDR-implantation technology it has unexpectedly been found that some of the selected antibodies comprised CDRs with canonical structures that are not normally found in the used framework. These antibodies are functional since they bind their antigen with high affinity (Example 1). Thus, using a single framework it is possible to create functional variability in antibody combining sites that is based on canonical loops that are atypical in a certain framework context. A library based on such a concept would have advantages over more conventional libraries since it can harbour antibodies with a wide variety of topologies and at the same time be highly efficient in the selected host system (*e.g. E. coli*).

Furthermore, the binding characteristics of antibodies could be improved using shuffling of selected CDRs in order to recombine the most optimal CDRs into a single antibody molecule. As will be appreciated, CDR-implantation technology permits shuffling of 1 to 6 CDRs at the same time and has been used on the basis of the library presented herein in the Examples to improve affinities of selected antibodies more than 30 times in a single step.

The present invention may therefore lead to novel combinations of classes of canonical structure, for example by combining canonical structures of classes that are not normally found in genes of the same germline family. For example, by incorporating CDRH2 CDRs into the CDRL2 position of a Vκ chain, variability from 4 classes of canonical structure can be accessed in this position, whereas in the natural Vκ antibody, there is only one class of canonical structure used in the CDRL2 position.

Preferably, the amplification primers are designed to amplify CDRs of a greater number of classes of canonical structure than the number of classes of canonical structure found in the germline gene family to which the master framework belongs, or CDRs of different classes of canonical structure from those found in the germline gene family to which the framework belongs. Predictions of the canonical structure adopted by a particular CDR may be determined using an online tool available at URL:
http://www.biochem.ucl.ac.uk/∼martin/abs/chothia.html.

The master framework need not be a naturally occurring one, but may for example have been optimised, *e.g.* for the expression or phage system to be used, or to reduce antigenicity *in vivo.*

The CDRs, having been amplified, may be subject to mutagenesis, *e.g.* using error-prone PCR, before being incorporated into the master framework (e.g. as described in WO98/32845), though this is not generally preferred since naturally occurring CDRs are less likely than artificial ones to be antigenic.

"Percent (%) nucleic acid sequence identity" is defined as the percentage of nucleic acid residues in a candidate sequence that are identical with the nucleic acid residues in the sequence with which it is being compared, after aligning the sequences and introducing gaps, if necessary, to achieve the maximum percent sequence identity, and not considering any conservative substitutions as part of the sequence identity. The percent identity values used herein were generated by the BLASTN module of WU-BLAST-2 (which was obtained from Altschul *et al.* (1996); URL: http://blast.wustl/edu/ blast/README.html). WU-BLAST-2 uses several search parameters, most of which are set to the default values. The adjustable parameters are set with the following values: overlap span =1, overlap fraction = 0.125. A percent nucleic acid sequence identity value is determined by the number of matching identical residues divided by the total number of residues of the "longer" sequence in the aligned region, multiplied by 100. The "longer" sequence is the one having the most actual residues in the aligned region (gaps introduced by WU-BLAST-2 to maximize the alignment score are ignored).

The following examples are provided for the better understanding of the invention, and make reference to the accompanying figures, in which:
**Figure 1 (parts A to D)** shows the incorporation of a CDRH2 loop from germline gene DP-29 into a framework of DP-47.
**Figure 2 (parts A to E)** shows the incorporation of a CDRH2 loop from germline gene DP-73 into a framework of DP-47.

### EXAMPLES

### Design of primers and assembly of antibody genes

Primers that are different from corresponding sequences in the DP-47 framework are used to amplify CDRs from different germline genes. In example 1A, the master framework and the framework of the gene from which the CDR is amplified (DP-29) are sufficiently similar that the thus-amplified sequence can be incorporated into a DP-47 framework without further modification. In example 1B, the frameworks are more dissimilar, and the thus-amplified sequence is further modified to make it more similar to the DP-47 framework before it is incorporated therein. This is achieved by use of primers that successively bring the framework regions that flank the CDRs into conformity with the selected framework in a designed and planned iterative process. In this way, it is possible to pick up CDR-loops that have canonical structures that are atypical of the selected DP-47 framework.

When, as here, it is desired to incorporate a specific CDR into the master framework, it can be advantageous to determine the homology (*i.e.* percentage identity) between the selected framework and the framework surrounding the atypical CDR to be incorporated into the selected framework. Of course, if one is using primers of a known sequence to "fish" for CDRs in a library, it is more important to determine the homology between the primers and the framework sequence.

The degree of homology determines the number of PCR amplification steps necessary to obtain the atypical CDR in the selected framework. This means that a lower degree of homology will result in several sequential PCR steps to convert the original FR flanking the atypical CDR into the sequence of the selected FR.

### Example 1A

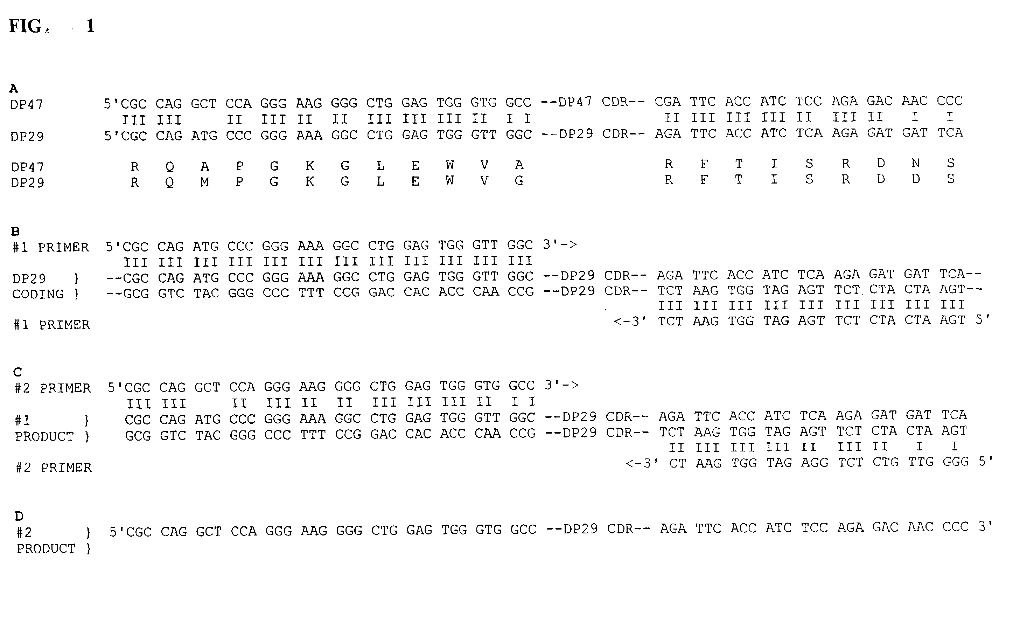

Figure 1 shows the sequences and steps involved in the amplification of DP-29 CDRH2, and its incorporation into nucleic acid encoding framework of DP-47.

Part A shows nucleic acid sequences encoding portions of the framework regions flanking the DP-47 and DP-29 CDRH2 loops and the deduced amino acid sequences. Nucleotide matches are denoted by the symbol I. As will be seen, there are some mismatches: 8 of 36 nucleotides and 7 of 27 nucleotides in the two flanking portions shown, respectively.

Part B shows amplification primers ("#1 primers") identical to the nucleic acid encoding portions of the framework regions flanking the DP-29 CDRH2 loops, aligned with the double-stranded DP-29 coding sequence.

Part C shows the amplification product ("#1 product") of the first PCR step (which was shown in Part B). Conditions for amplification are as for CDR amplification in WO98/32845. The #1 product is identical to the coding sequence of DP-29. Aligned with this are primers ("#2 primers") for a second PCR step. These are identical to the nucleic acid encoding corresponding portions of the framework regions flanking the DP-47 CDRH2 loops. Consequently the same mismatches are apparent as in part A.

Part D shows the product ("#2 product") of the second PCR step. This has the framework regions of DP-47 (the master framework) and the CDRH2 loop of DP-29.

Thus, there is sufficient sequence identity between the framework regions ofDP-47 and DP-29 flanking the CDRH2 loop for the loop to be switched from one framework to the other in a single PCR step.

The DP-29 germline gene encodes a CDRH2 of canonical class 4 (VBASE), whereas the CDRH2 of DP-47 is of canonical class 3 (VBASE).

The second PCR step could be performed as an overlap extension PCR step, since the primer used is identical to the master framework sequence into which the CDR is intended to be incorporated, for example using the conditions (and other primers) set out in WO98/32845.

### Example 1B

An iterative process of sequential PCR amplifications is used to insert a CDR into a DP-47 master framework from a germline gene (DP-73) which has significantly different sequences encoding the portions of the framework regions flanking the CDR. In this example the homology between the DP-47 V_{H} framework, adjacent to CDRH2, and the DP-73 framework is too low to allow for direct amplification (*e.g.* in an overlap extension PCR step) using primers wholly identical to DP-47. Thus, several individual PCR steps are used, each step using a unique primer pair. The primers are successively modified to become more homologous to the DP-47 primer.

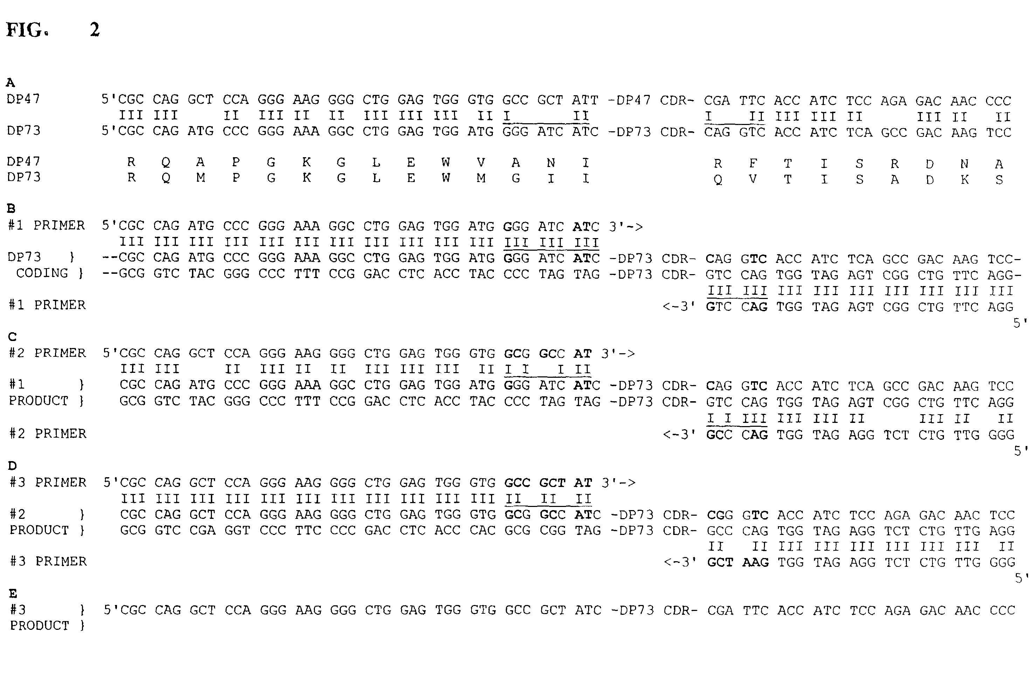

In this process it is important to carefully choose the proper distribution of the base modifications. Figure 2 shows this process. The underlined sequence is where the greatest differences occur between DP-47 and DP-73. Bold letters denote residues in the primers which are identical to those in DP-47, the master framework.

Parts A and B are analogous to the same parts of figure 1. Again, there are mismatches between the sequences encoding the portions of framework which flank the DP-47 and DP-73 CDRH2 loops, 13 mismatches out of 42 nucleotides and 9 mismatches out of 27 in the two flanking sequences, respectively.

In part C, instead of using primers identical to DP-47, primers which are chimaeras of DP-47 and DP-73 are used, to introduce changes into the framework regions of the amplified DP-73 fragment, to bring them partly into conformity with those of DP-47. So, rather than there being no mismatches between the primers and the DP-47 sequences (as in Example 1A), there are still some mismatches, though fewer than before, i.e. 2 in each flanking sequence.

Part D shows the amplification product ("#2 product") of the second PCR step, aligned with primers ("#3 primers") identical to corresponding portions of the DP-47 framework. As with example 1A, such primers could be used in overlap extension PCR. The third amplification step (analogously to the second in example 1A) leads to a fragment incorporating CDRH2 of DP-73 in a framework of DP-47.

In particular, in the second PCR step (which uses the #2 primers, shown in part C), the following base substitutions are made in the upper PCR primer relative to the #1 primer, used in the first PCR step (shown in part B): At position 35 (counted from the 5' end of the primer) G is changed to C; at position 37 A is changed to G and at position 38 T is changed to C. This results in a higher degree of homology than if a primer homologous to DP-47 was to be used directly in the second PCR step.

The intermediate PCR product from the second PCR step therefore still contains bases that are homologous to the DP73 sequence (G36 and C39). These bases will then not prime in the third PCR step, since the upper primer used in this step is 100% homologous to the DP47 sequence. However, bases 34, 37 and 38 of the amplification product ("#2 product") of the second amplification step are now homologous to the DP-47 sequence and this homology will give an annealing of 6 of 8 bases at the 3' end (underlined) of the upper primer in the third PCR step (shown in part D). This is to be compared to 3 out of 8 bases at the 3' end between DP47 and DP73. Such an increase in homology greatly facilitates the successful production of a DNA sequence comprising the DP-73 CDRH2 in the DP-47 framework.

Using the principles of this method any CDR can be transferred to any given and selected framework resulting in composite antibody molecules that possess combinations of natural CDR-loops and hence possibly also canonical structures, that can not be found in nature. Thus, combination of atypical but natural CDR-loops gives a basis for generation of an enormous variability in the antibody combining site and the created variants may be captured in large libraries using *e.g.* phage (Marks *et al.* 1991), ribosome (Hanes and Pluckthun, 1997) or covalent (WO98/37186) display technologies.

### References

Adkins JC, Spencer CM (1998) *Drugs* 56(4):619-26; discussion 627-8.

Al-Lazikani B, Lesk AM, Chothia C (1997) *J Mol Biol* 273(4):927-48

Chothia C, Lesk AM (1987) *J Mol Biol* 186:651-663

Chothia C *et al.* (1987) *J Mol Biol* 196:901-917

Chothia C *et al.* (1989) *Nature* 342:877-883

Chothia C, Lesk AM, Gherardi E, Tomlinson IM, Walter G, Marks JD, Llewelyn MB, Winter G (1992) *J Mol Biol* 227(3):799-817

D'haens G, Van Deventer S, Van Hogezand R, *et al.* (1999) *Gastroenterology* 116(5):1029-34

Duenas M, Borrebaeck CA (1994) *Biotechnology* (NY) 12(10):999-1002

Griffiths AD *et al.* (1993) *EMBO J* 12(2):725-734

Griffiths AD *et al.* (1994) *EMBO J* 13(14):3245-3260

Griffiths AD and Duncan AR (1998) *Curr Opin Biotechnol* 9:102-108

Hanes J and Pluckthun A (1997) *Proc Natl Acad Sci USA* 94(10):4937-42

Hoogenboom HR, Winter G (1992) *J Mol Biol* 227(2):381-8

Jirholt P, Ohlin M, Borrebaeck CAK, Soderlind E (1998) *Gene* 215(2):471-476

Kobayashi N, Soderlind E, Borrebaeck CAK (1997) *Biotechniques* 23(3):500-503

Lara-Ochoa F, Almagro JC, Vargas-Madrazo E, Conrad M (1996) *J Mol Evol* 43:678-684

Larrick JW, Danielsson L, Brenner Ca, *et al.* (1989) *Bio*/*Technology* 7:934

Malmborg AC, Duenas M, Ohlin M, *et al.* (1996) *J Immunol Methods* 198(1):51-7

Malmborg AC, Soderlind E, Frost L, Borrebaeck CA (1997) *J Mol Biol* 273(3):544-51

Marks JD, Hoogenboom HR, Bonnert TP, *et al.* (1991) *J Mol Biol* 222(3):581-597

McLaughlin P, White CA, Grillo-Lopez AJ, Maloney DG (1998) *Oncology* (Huntingt) 12(12): 1763-9; discussion 1769-70, 1775-7

Morea V, Tramontano A, Rustici M, Chothia C, Lesk AM (1998) *J Mol Biol* 275:269-294

Nissim A, Hoogenboom HR, Tomlinson IM, *et al.* (1994) *EMBO J* 13:692-698

Palares *et al.* (1999) *Exp. Clin. Immunogenet.* 16: 36-60

Pini A, Viti F, Santucci A, Carnemolla B, *et al.* (1998) *J Biol Chem* 273(34):21769-21776

Sheets MD, Amersdorfer P, Finnem R, *et al.* (1998) *Proc Natl Acad Sci USA* 95(11):6157-6162

Söderlind *et al.* (1999) *Immunotechnology* 4, 279-285

Tomlinson IM, Cox JP, Gherardi E, *et al.* (1995) *EMBO J* 14(18):4628-38

Vargas-Madrazo E, Lara-Ochoa F, Almagro JC (1995) *J Mol Biol* 254:497-504

Vaughan TJ, Williams AJ, Pritchard K, *et al.* (1996) *Nat Biotechnol* 14(3):309-314

Williamson RA, Burioni R, Sanna PP, *et al.* (1993) *Proc Natl Acad Sci USA* 90:4141-4145

Zebedee SL *et al.* (1992) *Proc Natl Acad Sci USA* 89:3175-3179

## Claims

1. A method for producing a polynucleotide sequence encoding an antibody variable domain, the variable domain comprising complementarity-determining regions (CDRs) located within a selected framework (the 'master framework'), the method comprising the steps of:
a) providing at least one nucleic acid molecule encoding one or more CDRs and associated framework regions (the 'original framework');
b) amplifying at least one CDR-encoding portion of the nucleic acid molecule(s) of step (a) using one or more pairs of oligonucleotides as amplification primers and;
c) assembling a polynucleotide sequence encoding an antibody variable domain by combining the amplified CDR-encoding nucleotide sequences produced in step (b) with nucleotide sequences encoding said master framework,
wherein the oligonucleotide primers of step (b) comprise nucleotide sequences which differ from the corresponding nucleotide sequences encoding said master framework.

2. A method for producing a library of polynucleotide sequences each encoding an antibody variable domain comprising complementarity-determining regions (CDRs) located within a common selected framework (the 'master framework'), the method comprising the steps of:
a) providing a population of nucleic acid molecules encoding one or more complementarity-determining regions (CDRs) and associated framework regions (the 'original framework');
b) amplifying at least one CDR-encoding portion of the nucleic acid molecules of step (a) using one or more pairs of oligonuclcotides as amplification primers and;
c) assembling a polynucleotide sequence encoding an antibody variable domain by combining the amplified CDR-encoding nucleotide sequences produced in step (b) with nucleotide sequences encoding said master framework,
wherein the oligonucleotide primers of step (b) comprise nucleotide sequences which differ from the corresponding nucleotide sequences encoding said master framework.

3. A method according to Claim 1 or 2 comprising the steps of:
i) providing at least one pair of oligonucleotides;
ii) using each said pair of oligonucleotides as amplification primers to amplify nucleotide sequences encoding different CDRs, and;
iii) assembling polynucleotide sequences encoding antibody variable domains by incorporating nucleotide sequences derived from step (ii) above with nucleotide sequences encoding said master framework,
wherein the oligonucleotides of step (i) have sequences which differ from corresponding sequences encoding said master framework.

4. A method according to Claim 1 or 2 wherein the polynucleotide sequence(s) assembled in step (c) encodes an immunoglobulin G (IgG) variable domain.

5. A method according to Claim 1 or 2 wherein the polynucleotide sequence(s) assembled in step (c) encodes an IgG heavy chain or light chain.

6. A method according to Claim 1 or 2 wherein the polynucleotide sequence(s) assembled in step (c) encodes a non-naturally occurring antibody variable domain.

7. A method according to Claim 1 or 2 wherein at least one of the polynucleotide sequences assembled in step (c) encodes an antibody variable domain comprising at least one CDR having a canonical structure which is atypical of CDRs in naturally-occurring antibody variable domains comprising the master framework.

8. A method according to Claim 1 or 2 wherein at least one of the polynucleotide sequences assembled in step (c) encodes an antibody variable domain comprising at least one CDR derived from a different germline gene family to that of the master framework.

9. A method according to 1 or 2 wherein step (a) comprises providing a population of nucleic acid molecules each encoding an antibody variable domain.

10. A method according to 9 wherein the nucleic acid molecules each encode an antibody variable domain from the same germline gene family.

11. A method according to Claim 9 wherein the oligonucleotide primer pairs of step (b) selectively hybridise to a target sub-population of nucleic acid molecules provided in step (a).

12. A method according to Claim 11 wherein the target sub-population of nucleic acid molecules each encode a antibody variable domain from the same germline gene family.

13. A method according to Claim 10 or 12 wherein the nucleic acid molecules each encode an antibody variable domain derived from the same germline gene.

14. A method according to Claim 13 wherein the germline gene is selected from the group consisting of DP-29 and DP-73.

15. A method according to Claim 1 or 2 wherein the master framework is derived from a germline gene selected from the group consisting of DP-47 and DPL-3.

16. A method according to any one of Claims 1 to 15 comprising a further step, performed after step (b) and prior to step (c), of modifying the nucleotide sequence of the amplified CDR-encoding molecules of step (b) such that the portions of said amplified molecules which encode framework regions share greater sequence identity with the corresponding portions of the master framework.

17. A method according to Claim 16 wherein the nucleotide sequence of the amplified CDR-encoding molecules of step (b) are modified such that the portions of said amplified molecules which encode framework regions share 100% sequence identity with the corresponding portions of the master framework.

18. A method according to Claim 16 or 17 wherein the further step comprises a single round of PCR amplification using oligonucleotide primers which comprise a nucleotide sequence which is a chimaera of the nucleotide sequences encoding the original and master frameworks.

19. A method according to Claim 16 or 17 wherein the further step comprises performing one or more additional rounds of PCR amplification of the CDR-encoding nucleic acid molecules produced in step b), each additional round of amplification being performed using oligonucleotide primers comprising a nucleotide sequence having an increasing number of nucleotide mismatches relative to the original framework sequence, those mismatches sharing sequence identity with the corresponding nucleotides of the master framework.

20. A method according to Claim 1 or 2 wherein step (c) comprises the use of overlap extension PCR.

21. A method according to Claim 1 or 2 comprising a further step of inserting the polynucleotide sequence(s) assembled in step (c) into an expression vector.

22. A method according to Claim 21 wherein the expression vector is a phagc display vector.

23. A method according to any one of Claims 1 to 22 further comprising the step of expressing the polynucleotide sequence(s) assembled in step (c) and screening the resultant polypeptide(s), comprising an antibody variable domain, for desired properties.

## Patentansprüche

1. Verfahren zur Erzeugung einer Polynucleotidsequenz, die für eine variable Antikörperdomäne codiert, wobei die variable Domäne *complementarity-determining regions* (CDRs) umfasst, die in einem ausgewählten Framework (dem "Master-Framework") lokalisiert sind, wobei das Verfahren die folgenden Schritte umfasst:
a) Bereitstellen wenigstens eines Nucleinsäuremoleküls, das für eine CDR oder mehrere CDRs und assoziierte Framework-Regionen (das "ursprüngliche Framework") codiert,
b) Amplifizieren wenigstens eines CDR-codierenden Abschnittes des Nucleinsãuremoleküls/der Nucleinsäuremoleküle des Schrittes (a) unter Verwendung eines Oligonucleotidpaares oder mehrerer Oligonucleotidpaare als Primer für die Amplifizierung, und
c) Zusammensetzen einer Polynucleotidsequenz, die für eine variable Antikörperdomäne codiert, durch das Kombinieren der amplifizierten, CDR-codierenden Nucleotidsequenzen, die im Schritt (b) erzeugt wurden, mit Nucleotidsequenzen, die für das genannte Master-Framework codieren,
wobei die Oligonucleotidprimer aus Schritt (b) Nucleotidsequenzen umfassen, die sich von den entsprechenden Oligonucleotidsequenzen unterscheiden, die für das genannte Master-Framework codieren.

2. Verfahren zur Erzeugung einer Bibliothek von Polynucleotidsequenzen, von denen jede für eine variable Antikörperdomäne codiert, die *complementarity-de* *termining regions* (CDRs) umfasst, die in einem gemeinsamen ausgewählten Framework (dem "Master Framework") lokalisiert sind, wobei das Verfahren die folgenden Schritte umfasst:
a) Bereitstellen einer Population von Nucleinsäuremolekülen, die für eine oder mehrere *complementarity-determining region(s)* (CDR(s)) und assoziierte Framework-Regionen (das "ursprüngliche Framework") codieren,
b) Amplifizieren wenigstens eines CDR-codierenden Abschnittes der Nucleinsäuremoleküle des Schrittes (a) unter Verwendung eines Oligonucleotidpaares oder mehrerer Oligonucleotidpaare als Primer für die Amplifizierung, und
d) Zusammensetzen einer Polynucleotidsequenz, die für eine variable Antikörperdomäne codiert, durch das Kombinieren der amplifizierten, CDR-codierenden Nucleotidsequenzen, die im Schritt (b) erzeugt wurden, mit Nucleotidsequenzen, die für das genannte Master-Framework codieren,
wobei die Oligonucleotidprimer aus Schritt (b) Nucleotidsequenzen umfassen, die sich von den entsprechenden Oligonucleotidsequenzen unterscheiden, die für das genannte Master-Framework codieren.

3. Verfahren gemäß Anspruch 1 oder 2, das die folgenden Schritte umfasst:
i) Bereitstellen wenigstens eines Oligonucleotidpaares,
ii) Verwendung jedes der genannten Oligonucleotidpaare als Primer für die Amplifizierung von Nucleotidsequenzen, die für unterschiedliche CDRs codieren, und
iii) Zusammensetzen von Polynucleotidsequenzen, die für variable Antikörperdomänen codieren, durch das Inkorporieren von Nucleotidsequenzen, die aus dem obigen Schritt (ii) stammen, in Nucleotidsequenzen, die für das genannte Master-Framework codieren,
wobei die Oligonucleotide aus dem Schritt (i) Sequenzen aufweisen, die sich von entsprechenden Sequenzen unterscheiden, die für das genannte Master-Framework codieren.

4. Verfahren gemäß Anspruch 1 oder 2, wobei die im Schritt (c) zusammengesetzte(n) Polynucleotidsequenz(en) für eine variable Domäne eines Immunglobulins G (IgG) codiert bzw. codieren.

5. Verfahren gemäß Anspruch 1 oder 2, wobei die im Schritt (c) zusammengesetzte(n) Polynucleotidsequenz(en) für eine schwere Kette oder eine leichte Kette eines IgG codiert bzw. codieren.

6. Verfahren gemäß Anspruch 1 oder 2, wobei die im Schritt (c) zusammengesetzte(n) Polynucleotidsequenz(en) für eine nicht natürlich vorkommende variable Antikörperdomäne codiert bzw. codieren.

7. Verfahren gemäß Anspruch 1 oder 2, wobei wenigstens eine der im Schritt (c) zusammengesetzten Polynucleotidsequenzen für eine variable Antikörperdomäne codiert, die wenigstens eine CDR umfasst, die eine kanonische Struktur hat, die für CDRs in natürlich vorkommenden variablen Antikörperdomänen, die das Master-Framework umfassen, untypisch ist.

8. Verfahren gemäß Anspruch 1 oder 2, wobei wenigstens eine der im Schritt (c) zusammengesetzten Polynucleotidsequenzen für eine variable Antikörperdomäne codiert, die wenigstens eine CDR umfasst, die von einer anderen Keimbahn-Genfamilie als derjenigen des Master-Frameworks abstammt.

9. Verfahren gemäß 1 oder 2, wobei der Schritt (a) das Bereitstellen einer Population von Nucleinsäuremolekülen umfasst, von denen jedes für eine variable Antikörperdomäne codiert.

10. Verfahren gemäß 9, wobei jedes der Nucleinsäuremoleküle für eine variable Antikörperdomäne aus der gleichen Keimbahn-Genfamilie codiert.

11. Verfahren gemäß Anspruch 9, wobei die Paare der Oligonucleotidprimer aus Schritt (b) selektiv mit einer Ziel-Subpopulation von Nucleinsäuremolekülen hybridisieren, die im Schritt (a) bereit gestellt wurden.

12. Verfahren gemäß Anspruch 11, wobei jedes Nucleinsäuremolekül der Ziel-Subpopulation für eine variable Antikörperdomäne aus der gleichen Keimbahn-Genfamilie codiert.

13. Verfahren gemäß Anspruch 10 oder 12, wobei jedes Nucleinsäuremolekül für eine variable Antikörperdomäne codiert, die vom gleichen Keimbahn-Gen abstammt.

14. Verfahren gemäß Anspruch 13, wobei das Keimbahn-Gen aus der Gruppe ausgewählt ist, die aus DP-29 und DP-73 besteht.

15. Verfahren gemäß Anspruch 1 oder 2, wobei das Master-Framework von einem Keimbahn-Gen abstammt, das aus der Gruppe ausgewählt ist, die aus DP-47 und DPL-3 besteht.

16. Verfahren gemäß einem beliebigen der Ansprüche 1 bis 15, das einen weiteren Schritt, der nach dem Schritt (b) und vor dem Schritt (c) durchgeführt wird, des Modifizierens der Nucleotidsequenz der amplifizierten CDR-codierenden Moleküle aus dem Schritt (b) umfasst, so dass die Abschnitte der genannten amplifizierten Moleküle, die für Framework-Regionen codieren, eine größere Sequenzübereinstimmung mit den entsprechenden Abschnitten des Master-Frameworks zeigen.

17. Verfahren gemäß Anspruch 16, wobei die Nucleotidsequenzen der amplifizierten CDR-codierenden Moleküle aus dem Schritt (b) so modifiziert werden, dass dle Abschnitte der genannten amplifizierten Moleküle, die für Framework-Regionen codieren, eine 100%-ige Sequenzübereinstimmung mit den entsprechenden Abschnitten des Master-Frameworks aufweisen.

18. Verfahren gemäß Anspruch 16 oder 17, wobei der weitere Schritt eine einzige Runde einer PCR-Amplifizierung unter Verwendung von Oligonucleotidprimem umfasst, die eine Nucleotidsequenz umfassen, die eine Schimäre der Nucleotidsequenzen ist, die für das ursprüngliche Framework und das Master-Framework codieren.

19. Verfahren gemäß Anspruch 16 oder 17, wobei der weitere Schritt das Durchführen einer oder mehrerer zusätzlichen Runde(n) einer PCR-Amplifizierung der CDR-codierenden Nucleinsäuremoleküle umfasst, die im Schritt (b) erzeugt wurden, wobei jede zusätzliche Amplifizierungsrunde unter Verwendung von Oligonucleotidprimem durchgeführt wird, die eine Nucleotidsequenz umfassen, die eine zunehmende Anzahl von Nucleotid-Fehlpaarungen gegenüber der ursprünglichen Framework-Sequenz aufweist, wobei diese Fehlpaarungen eine Sequenzübereinstimmung mit den entsprechenden Nucleotiden des Master-Frameworks aufweisen.

20. Verfahren gemäß Anspruch 1 oder 2, wobei der Schritt (c) den Einsatz einer Overlap-Extension-PCR umfasst.

21. Verfahren gemäß Anspruch 1 oder 2, das einen weiteren Schritt aus dem Einfügen der Polynucleotidsequenz(en), die im Schritt (c) zusammengesetzt wurde(n), in einen Expressionsvektor umfasst.

22. Verfahren gemäß Anspruch 21, wobei der Expressionsvektor ein Phage-Display-Vektor ist.

23. Verfahren gemäß einem beliebigen der Ansprüche 1 bis 22, das ferner den Schritt der Expression der Polynucleotidsequenz(en), die im Schritt (c) zusammengesetzt wurde(n), und das Screenen des resultierenden Polypeptids bzw. der resultierenden Polypeptide, das eine variable Antikörperdomäne aufweist bzw. die eine variable Antikörperdomäne aufweisen, bezüglich gewünschter Eigenschaften umfasst.

## Revendications

1. Procédé de production d'une séquence de polynucléotide codant pour un domaine variable d'anticorps, le domaine variable comprenant des régions de détermination de la complémentarité (RDC) situées au sein d'une infrastructure choisie (« l'infrastructure maîtresse »), le procédé comprenant les étapes consistant à :
a) fournir au moins une molécule d'acide nucléique codant pour une ou plusieurs RDC et régions à infrastructure associée (« l'infrastructure d'origine ») ;
b) amplifier au moins un segment codant pour une RDC de la ou des molécule(s) d'acides nucléiques de l'étape (a) en utilisant une ou plusieurs paires d'oligonucléotides comme amorces d'amplification et ;
c) assembler une séquence de polynucléotides codant pour un domaine variable d'anticorps par combinaison des séquences de nucléotides codant pour les RDC amplifiées produites à l'étape (b) aux séquences de nucléotides codant pour ladite infrastructure maîtresse,
dans lequel les amorces oligonucléotidiques de l'étape (b) comprennent des séquences de nucléotides qui diffèrent des séquences de nucléotides correspondantes codant pour ladite infrastructure maîtresse.

2. Procédé de production d'une collection de fragments de séquences de polynucléotides chacune codant pour un domaine variable d'anticorps comprenant des régions de détermination de la complémentarité (RDC) situées au sein d'une infrastructure commune choisie (« l'infrastructure maîtresse »), le procédé comprenant les étapes consistant à :
a) fournir une population de molécules d'acides nucléiques codant pour une ou plusieurs régions de détermination de la complémentarité (RDC) et régions à infrastructure associée (« l'infrastructure d'origine ») ;
b) amplifier au moins un segment codant pour une RDC des molécules d'acides nucléiques de l'étape (a) en utilisant une ou plusieurs paires d'oligonucléotides comme amorces d'amplification et ;
c) assembler une séquence de polynucléotides codant pour un domaine variable d'anticorps par combinaison des séquences de nucléotides codant pour les RDC amplifiées produites à l'étape (b) aux séquences de nucléotides codant pour ladite infrastructure maîtresse,
dans lequel les amorces oligonucléotidiques de l'étape (b) comprennent des séquences de nucléotides qui diffèrent des séquences de nucléotides correspondantes codant pour ladite infrastructure maîtresse.

3. Procédé selon la revendication 1 ou la revendication 2 comprenant les étapes consistant à :
i) fournir au moins une paire d'oligonucléotides ;
ii) utiliser chaque dite paire d'oligonucléotides comme amorce d'amplification pour amplifier les séquences de nucléotides codant pour différentes RDC, et ;
iii) assembler les séquences de polynucléotides codant pour des domaines variables d'anticorps par incorporation des séquences de nucléotides dérivées de l'étape (ii) ci-dessus comprenant les séquences de nucléotides codant pour ladite infrastructure maîtresse,
dans lequel les oligonucléotides de l'étape (i) possèdent des séquences qui diffèrent des séquences correspondantes codant pour ladite infrastructure maîtresse.

4. Procédé selon la revendication 1 ou la revendication 2, dans lequel la ou les séquence(s) de polynucléotides assemblée(s) à l'étape (c) code(nt) pour un domaine variable d'immunoglobuline G (IgG).

5. Procédé selon la revendication 1 ou la revendication 2, dans lequel la ou les séquence (s) de polynucléotides assemblée(s) à l'étape (c) code(nt) pour une chaîne lourde ou chaîne légère d'IgG.

6. Procédé selon la revendication 1 ou la revendication 2, dans lequel la ou les séquence(s) de polynucléotides assemblée(s) à l'étape (c) code(nt) pour un domaine variable d'anticorps d'origine non naturelle.

7. Procédé selon la revendication 1 ou la revendication 2, dans lequel au moins une des séquences de polynucléotides assemblées à l'étape (c) code pour un domaine variable d'anticorps comprenant au moins une RDC possédant une infrastructure canonique qui est atypique des RDC dans les domaines variables d'anticorps d'origine naturelle comprenant l'infrastructure maîtresse.

8. Procédé selon la revendication 1 ou la revendication 2, dans lequel au moins une des séquences de polynucléotides assemblées à l'étape (c) code pour un domaine variable d'anticorps comprenant au moins une RDC dérivée d'une famille de gènes d'une lignée germinale différente de celle de l'infrastructure maîtresse.

9. Procédé selon la revendication 1 ou la revendication 2, dans lequel l'étape (a) comprend la fourniture d'une population de molécules d'acides nucléiques chacune codant pour un domaine variable d'anticorps.

10. Procédé selon la revendication 9, dans lequel les molécules d'acides nucléiques codent chacune pour un domaine variable d'anticorps à partir d'une famille de gènes de la même lignée germinale.

11. Procédé selon la revendication 9, dans lequel les paires d'amorces oligonucléotidiques de l'étape (b) s'hybrident sélectivement à une sous population cible de molécules d'acides nucléiques fournie à l'étape (a).

12. Procédé selon la revendication 11, dans lequel la sous population cible de molécules d'acides nucléiques code chacune pour un domaine variable d'anticorps à partir de la famille de gènes d'une même lignée germinale.

13. Procédé selon la revendication 10 ou la revendication 12, dans lequel les molécules d'acides nucléiques codent chacune pour un domaine variable d'anticorps dérivé du gène de la même lignée germinale.

14. Procédé selon la revendication 13, dans lequel le gène de la lignée germinale est choisi dans le groupe constitué de DP-29 et DP-73.

15. Procédé selon la revendication 1 ou la revendication 2, dans lequel l'infrastructure maîtresse est dérivée d'un gène d'une lignée germinale choisi dans le groupe constitué de DP-47 et DPL-3.

16. Procédé selon l'une quelconque des revendications 1 à 15, comprenant une étape supplémentaire, exécutée après l'étape (b) et préalablement à l'étape (c), de modification de la séquence de nucléotides des molécules codant pour les RDC amplifiées de l'étape (b) de sorte que les segments desdites molécules amplifiées qui codent pour les régions de l'infrastructure partagent une identité de séquence supérieure avec les segments correspondants de l'infrastructure maîtresse.

17. Procédé selon la revendication 16, dans lequel les séquences de nucléotides des molécules codant pour les RDC amplifiées de l'étape (b) sont modifiées de sorte que les segments desdites molécules amplifiées qui codent pour les régions de l'infrastructure partagent 100 % d'identité de séquence avec les segments correspondants de l'infrastructure maîtresse.

18. Procédé selon la revendication 16 ou la revendication 17, dans lequel l'étape supplémentaire comprend un seul passage de l'amplification en chaîne par polymérase (PCR) en utilisant des amorces oligonucléotidiques qui comprennent une séquence de nucléotides qui est une chimère des séquences de nucléotides codant pour les infrastructures d'origine et maîtresse.

19. Procédé selon la revendication 16 ou la revendication 17, dans lequel l'étape supplémentaire comprend l'exécution d'un ou plusieurs passages supplémentaires de l'amplification en chaîne par polymérase (PCR) des molécules d'acides nucléiques codant pour les RDC produites à l'étape (b), chaque passage supplémentaire d'amplification étant exécuté en utilisant des amorces oligonucléotidiques comprenant une séquence de nucléotides ayant un nombre croissant d'incompatibilités des nucléotides relativement à la séquence de l'infrastructure d'origine, ces incompatibilités partageant une identité de séquence avec les nucléotides correspondants de l'infrastructure maîtresse.

20. Procédé selon la revendication 1 ou la revendication 2, dans lequel l'étape (c) comprend l'utilisation de l'amplification en chaîne par polymérase (PCR) d'extension de chevauchement.

21. Procédé selon la revendication 1 ou la revendication 2 comprenant une étape supplémentaire d'insertion de la ou des séquence(s) de polynucléotides assemblée(s) à l'étape (c) dans un vecteur d'expression.

22. Procédé selon la revendication 21, dans lequel le vecteur d'expression est un vecteur de présentation des phages.

23. Procédé selon l'une quelconque des revendications 1 à 22 comprenant en outre l'étape d'expression de la ou des séquence(s) de polynucléotides assemblée(s) à l'étape (c) et de criblage du ou des polypeptide(s) résultant(s), comprenant un domaine variable d'anticorps, pour obtenir les propriétés désirées.
